# EUROPEAN PATENT SPECIFICATION

(11) **EP 4 359 515 B1**
(45) Date of publication and mention of the grant of the patent: **25.12.2024**
(21) Application number: 22735151.7
(22) Date of filing: 20.06.2022
(51) Int. Cl.: A61K 39/12, C12N 1/36, A61L 9/20, A61K 39/00, A61N 5/06

(54) **IN-SITU AND REAL-TIME GENERATION AND ADMINISTRATION OF VIRAL VACCINES USING UV LIGHT INACTIVATION**
IN-SITU- UND ECHTZEITERZEUGUNG UND -VERABREICHUNG VON VIRALEN IMPFSTOFFEN MITTELS UV-LICHT-INAKTIVIERUNG
GÉNÉRATION ET ADMINISTRATION IN SITU ET EN TEMPS RÉEL DE VACCINS VIRAUX PAR INACTIVATION DE LA LUMIÈRE UV

(30) Priority: 24.06.2021 EP 21181310
(43) Date of publication of application: 01.05.2024
(73) Proprietor: Signify Holding B.V., 5656 AE Eindhoven (NL)
(72) Inventor: DE SAMBER, Marc, Andre, 5656 AE Eindhoven (NL)
(74) Representative: Verweij, Petronella Daniëlle
(86) International application number: PCT/EP2022/066730
(87) International publication number: WO 2022/268720

(56) References cited:
- EP-A1- 1 496 114
- WO-A1-2016/196904
- WO-A2-2007/035907
- US-A1- 2006 045 796
- US-A1- 2017 028 089
- US-A1- 2017 121 701
- US-A1- 2017 304 472
- US-A1- 2019 100 744
- HAJI MALAYERI ADEL ET AL: "Fluence (UV Dose) Required to Achieve Incremental Log Inactivation of Bacteria, Protozoa, Viruses and Algae Revised, updated and expanded by", 21 May 2015 (2015-05-21), XP055870078, Retrieved from the Internet <URL:https://uvsolutionsmag.com/stories/pdf/archives/180301_UVSensitivityReview_full.pdf> [retrieved on 20211207]
- WELCH DAVID ET AL: "FAR-UVC LIGHT: A NEW TOOL TO CONTROL THE SPREAD OF AIRBORNE MEDIATED MICROBIAL DISEASES", vol. 8, no. 2752, 1 December 2018 (2018-12-01), pages 1 - 7, XP055842121, Retrieved from the Internet <URL:https://www.nature.com/articles/s41598-018-21058-w.pdf> DOI: 10.1038/s41598-018-21058-w
- BHARDWAJ SANJEEV K. ET AL: "UVC-based photoinactivation as an efficient tool to control the transmission of coronaviruses", SCIENCE OF THE TOTAL ENVIRONMENT, vol. 792, 16 June 2021 (2021-06-16), AMSTERDAM, NL, pages 148548, XP055870092, ISSN: 0048-9697, DOI: 10.1016/j.scitotenv.2021.148548
- SODIQ AHMED ET AL: "Addressing COVID-19 contagion through the HVAC systems by reviewing indoor airborne nature of infectious microbes: Will an innovative air recirculation concept provide a practical solution?", ENVIRONMENTAL RESEARCH, ACADEMIC PRESS, SAN DIEGO, CA, US, vol. 199, 15 May 2021 (2021-05-15), XP086611879, ISSN: 0013-9351, [retrieved on 20210515], DOI: 10.1016/J.ENVRES.2021.111329

## Description

### FIELD OF THE INVENTION

The invention relates to a system for distributing an attenuated airborne pathogen. The invention also relates to an arrangement comprising an indoor space and the system. The invention further relates to a method for distributing an attenuated airborne pathogen.

### BACKGROUND OF THE INVENTION

Pathogen attenuation is known in the art. For instance, Indian patent application 202021056592 describes a device for attenuating pathogens, comprising a closed air pathway with air inlet and air outlet, a variable speed fan to flow air in path of the air pathway, a variable intensity germicidal light source to irradiate air in the air pathway, an electronic control circuit connected to the fan to control the speed and the electronic control circuit connected to the germicidal light source to control the intensity, wherein the device irradiates air in the air pathway with controlled radiation which inhibits the ability of at least one infectious organism to multiply in human body keeping the antigen layer of the infectious organism recognizable by the self-defense system of the human body to produce anti bodies. The pathogen is partially attenuated. The intensity of the germicidal light source is controllable. The speed of the fan is controllable. There are germicidal light sources of multiple wavelengths.

WO2007/035907A discloses a light source that includes a radiation source capable of producing radiation at a wavelength that can deactivate pathogens. A housing contains the radiation source and includes a visible light transmitting component that is coated with a phosphor capable of producing visible light when excited. The housing includes multiple openings that allow air to flow through the interior of the light source where it is exposed to the germicidal radiation. The radiation source includes an enclosure having a quartz component capable of absorbing radiation having a wavelength of 185 nm, which can create ozone. Additionally, the light source includes a blower that increases the airflow through the light source. The light source can be tubular to approximate a traditional fluorescent light, or it can include a threaded base so that it can be coupled to an incandescent light fixture.

D. Welch et. al., "Far-UVC light: a new tool to control the spread of airborne mediated microbial diseases", Scientific reports, Vol. 8, no 2752 (2018), discloses an approach to UV-based sterilization using single-wavelength far-UVC light generated by filtered excimer lamps, which selectively inactivate microorganisms, but does not produce biological damage to exposed mammalian cells and tissues. The approach is based on biophysical principles in that far-UVC light can traverse and therefore inactivate bacteria and viruses which are typically micrometer dimensions or smaller, whereas due to its strong absorbance in biological materials, far-UVC light cannot penetrate even the outer dead-cell layers of human skin, nor the outer tear layer on the surface of the eye.

WO2016/196904A discloses an apparatus for generating at least one radiation for selectively killing viruses. A first radiation source arrangement can be provided which is configured to generate at least one radiation having one or more wavelengths provided in a range of about 200 nm to about 230 nm, and at least one second arrangement can be provided which is configured to prevent the at least one radiation from having any wavelength that is outside of the range can be provided or which can be substantially harmful to cells of the body.

US2017/304473A discloses a portable ultraviolet UV device for UV disinfection and sterilization of a container, a room, a space or a defined environment. The container is selected from the group consisting of: (A) a container for fermenting an alcoholic beverage; (B) a container for storing or transporting a dairy product, a liquid dairy, a liquid dairy composition or a dry dairy composition; (C) a container for water, milk, coffee, tea, juice, or a carbonated beverage; and (D) a container for a biological fluid.

US2017/028089A discloses a device for sterilizing microorganisms on a liquid or solid substrate. The device includes a light source for producing a light and an optical device positioned proximate the light source. The optical device is configured to focus the light generated by the light source to provide a high intensity light output. The optical device also includes a dichroic reflector. The dichroic reflector is configured to pass thermal energy generated by the light source and reflect the light produced by the light source. The device also includes a power supply, where the power supply is coupled to the light source and the optical device. The device kills microbial organisms presented within the range of the high intensity light output.

US2019/100744A discloses the use of a high energy, low heat UV source which rapidly kills cells without damaging structures necessary for the production of an antibody response. It is discovered that such a process takes mere seconds rather than an hour or more, leading to rapid production of vaccines from live cell cultures. Such cells can then be introduced into patients as vaccines, or to stimulate additional antibody production in an already infected individual.

EP1496114 discloses directly contacting a liquid medium that contains microorganisms with a light source that emits ultraviolet (UV) radiation for generating vaccines.

US2017/121701 discloses an approach for preparing a vaccine using ultraviolet radiation that involves multiple iterations of inactivation of the vaccine using an ultraviolet radiation source at a set of different wavelengths and dosages. A recognition test of the vaccine using the set of different wavelengths and dosages is performed after the multiple iterations of inactivation. A controller compares results from the inactivation test and the recognition test to determine an area of acceptable radiation dosages and wavelengths generated from the ultraviolet radiation source that irradiate the live organisms without affecting efficacy and safety of the vaccine.

US2006/045796 discloses a method for determining an effective dose of monochromatic or polychromatic light from one or more light sources to inactivate microorganisms present in a biological fluid in a flow-through-reactor with one or more thermostatic light sources.

S.K. Bhardwaj et. al., "UVC-based photoinactivation as an efficient tool to control the transmission of coronaviruses", Science of the total environment, vol. 792 (2021), discloses environmental disinfection and sanitation in indoor areas, hospitals, and clinical rooms by using UVC irradiation of high energy and short wavelengths in the 200- 290 nm range which possesses great potential for germicidal disinfection. The properties of this UVC light allow to damage or destruct the nucleic acids (DNA/RNA) in diverse microbes ( e.g., bacteria, fungi, and viruses).

A. Sodiq et. al., "Addressing COVID-19 contagion through the HVAC system by reviewing indoor airborne nature of infectious microbes: will an innovative air recirculation concept provide a practical solution", Environmental research, academic press, San Diego, CA, US, Vol. 199 (2021), discloses to use UV lamps in HVAC systems for prevent spreading of viruses in indoor spaces.

### SUMMARY OF THE INVENTION

The recent and pandemic outbreak of COVID-19 (a human-to-human transmitted Corona virus) is a warning that more powerful methods and systems to fight and monitor infectious diseases may be needed.

The transmission of many infectious diseases, including COVID-19, can occur via contaminated persons, that spread infectious material via indirect transmission on surfaces or via airborne particles that carry the infectious material and may be inhaled by susceptible people.

There is a universal and widely acknowledged need for a vaccine against severe acute respiratory syndrome coronavirus 2 (SARS-CoV-2) because of its widespread circulation and high mortality and morbidity and the ongoing pandemic. Massive worldwide efforts to generate and fabricate a vaccine has led to many candidate vaccines and at this time first vaccines are cleared for human use.

The basic construction of the SARS-CoV-2 virus may be well understood. On the surface of the virus are 2 proteins: the spike protein and the membrane protein. Within the virus particle is the nuclear protein. The need for continuous alertness and fast provision of countermeasures in terms of infection prevention (including vaccination) and cure (therapeutics) may be vital. Desirable, such methods may serve the whole world population including less developed areas, leading to the need for low-cost fabrication and easy administration methods. Developing vaccines, however, is time consuming and costly, and may encounter new challenges with new pathogens.

The prior art may further describe the use of attenuated vaccines, such as attenuated viruses, for vaccination. Attenuated vaccines may be provided by producing large quantities of a pathogen, but reducing the virulence of the pathogen, such as by "evolving" the pathogen in the presence of (cells of) a different host organism, or by exposing the pathogen to a heat treatment. However, attenuated vaccines may still be time consuming to develop and costly to provide, and may fail to illicit an appropriate immune response, such as when the antigens are modified as a result of the attenuation.

Further, vaccines may be thermally instable, which may negatively impact the storage duration of the vaccine, and may need to be addressed during shipping, which may complicate logistics.

Further, the prior art may describe closed disinfection/attenuation systems, which may inherently lead to a limitation in power/dose/processed air because of geometrical limitations.

Hence, it is an aspect of the invention to provide an alternative system and/or an alternative method and/or an alternative arrangement with an indoor space, which preferably further at least partly obviate one or more of above-described drawbacks. The present invention may have as object to overcome or ameliorate at least one of the disadvantages of the prior art, or to provide a useful alternative.

Hence, in a first aspect, the invention provides a system for distributing an attenuated airborne pathogen (or "pathogen") in an indoor space (especially an indoor space for humans or animals). Especially, the system may be configured to (a) subject in a treatment mode first air comprising an airborne pathogen to first radiation to attenuate the airborne pathogen to provide second air with the attenuated airborne pathogen. Further, the system may be configured to (b) introduce the second air (with the attenuated airborne pathogen) in the indoor space. Especially, in embodiments a spectral power distribution of the first radiation may be controllable. More especially, in embodiments the spectral power distribution of the first radiation may be controllable in at least part of a first wavelength range of 100-380 nm. Further, the system may comprise a control system. In embodiments, the control system may be configured to control the treatment mode. Alternatively or additionally, in embodiments the control system may be configured to control the spectral power distribution of the first radiation. More especially, the control system may be configured to control the spectral power distribution of the first radiation of the treatment mode. Yet, even more especially the control system may be configured to control the spectral power distribution of the first radiation of the treatment mode in dependence of the airborne pathogen to be attenuated.

Hence, in specific embodiments the invention provides a system for distributing an attenuated airborne pathogen in an indoor space for humans or animals, wherein the system is configured to (a) subject in a treatment mode first air comprising an airborne pathogen to first radiation to attenuate the airborne pathogen to provide second air with the attenuated airborne pathogen, and to (b) introduce the second air (with the attenuated airborne pathogen) in the indoor space; wherein a spectral power distribution of the first radiation is controllable in at least part of a first wavelength range of 100-380 nm, wherein the system comprises a control system configured to control the spectral power distribution of the first radiation of the treatment mode in dependence of the airborne pathogen to be attenuated.

With such system it may e.g. be possible to selectively deactivate the RNA- or DNA-based reproduction capacity/infectivity of a pathogen, especially of an airborne and respiratory disease pathogen (e.g., the SARS-CoV-2 virus, the TB bacteria, etc.), using light as actuation method, with the purpose to use the attenuated pathogen (such as an attenuated virus) in situ for inducing immunological responses in target persons or animals, aiming at creating an immune protection (also known as 'vaccination'). The introduction of the attenuated viral load into the body of the target person (or animal) may happen via natural inhalation, and next the response may be induced in the endothelial cell liner in the respiratory track organs. The actuation light may in embodiments be characterized by the use of one or more wavelengths that are selected such that (mainly) polynucleotides are disrupted, while maintaining recognition (antigen) proteins of the pathogen (such as e.g., S-proteins on a virus), and hence maintaining the ability to induce an immunological response in a host/target person (or animal). For instance, based on the understanding of the pathogen (its molecular composition), the pathogen load in the air, and the environmental conditions the pathogen resides in/is embedded in, the wavelength type(s), doses (energy x time) and wavelength setting ratios (e.g., a ratio of 260 and 220 nm light) may be selected as to provide an (essentially) full deactivation of the polynucleotide (RNA or DNA) combined with a minimal/limited damage to the remaining immune-inducing parts of the pathogen ('dead' virus components). It may e.g. be possible to choose specific settings for the light type/conditions based on historical data (from scientific studies on the same or a comparable pathogen), or from pathogen molecular analysis followed by modelling of the optical properties (absorption characteristics) of the pathogen (with its constituting chromophore sites). This may facilitate determining a pathogen-specific absorption spectrum of pathogen molecules, which may enable specifically targeting individual type chromophores in the molecule (such as targeting the mentioned polynucleotides versus proteins). Other parameters that may guide the choice of the light wavelengths and its settings may be the adjacent effects in the optical properties of the pathogen air matrix (the pathogen in its micro-environment) such as e.g., the embedding in light-shielding mucus or saliva molecules or physical-optical influencing properties such as e.g., droplet/particle size. Next to that, the air may contain other (non-pathogen) high level influencing parameters such as e.g., light-absorbing airborne molecules and particles. The size of the airborne particles may be an indication of its origin (nasal, throat, upper airways, lungs). In addition, the composition of the airborne particles may change depending on its origin. In general, the size of such airborne particles could be measured by means of a sensor/detector. The size of emitted particles and/or its origin might also be deduced from sound analysis (e.g., coughing, or sneezing sounds), using a sound sensor or sound detector. Hence, amongst others the invention provides in embodiments a method to deactivate specifically and selectively a pathogen (e.g., SARS-CoV-2 virus) in situ and real time as originating from a selected infectious person (super spreader) or groups of persons, and (sequentially) provide the modified viral load (with deactivated polynucleotide) to host/target persons with the purpose to induce an immunological response in the persons as to make these immune (immune-responsive) against invasion of infectious (life) viruses of the same type. Further, the invention may provide an in-site and real-time generation and administration of (protein-based) viral vaccines using UV light inactivation.

Further, the system of the invention may not require highly complex vaccination preparation methods (which may include a sequence of host cell culturing, isolation/purification, inactivation, etc.), but may rather be suitable for mass vaccination, such as in conditions where the logistics of the vaccination (transport, storage) may be difficult or quasi-impossible, such as in rural and difficult to access areas.

As indicated above, the invention provides a system for distributing an attenuated airborne pathogen (or "pathogen") in an indoor space (or "exposure room"), especially an indoor space for humans and/or animals, such as for humans, or such as for animals. In specific embodiments, the indoor space may be an indoor space for one or more of poultry and livestock.

Hence, the pathogen may especially be an airborne pathogen, i.e., a pathogen with airborne transmission. In particular, the (airborne) pathogen may spread through small particulates transmitted through air over time and distance.

The term "pathogen" may herein refer to a pathogen selected from the group comprising (disease-causing) bacteria, archaea, fungi, algae, protozoa, and viruses, including both DNA and RNA viruses, especially to one or more of bacteria, fungi and viruses.

Especially, the airborne pathogen comprises one or more of a virus, bacteria, and fungi. Especially, in embodiments the airborne pathogen may comprise a virus. In embodiments, the virus may comprise a corona virus, like severe acute respiratory syndrome-related coronavirus (SARS-CoV), causing the COVID-19 disease. Especially relevant viruses in the context of this invention for humans may be one or more of SARS-CoV-2 (Corona virus), Influenza A (flu), Rhinovirus (common cold), viral pneumonia, Swine Flu (H1N1) in humans, and also zoonotic diseases such as LPAI (low pathogenic avian flu) H7N9 and HPAI (high pathogenic avian flu) H5N1 in human, but also bacteria such as *Mycobacterium tuberculosis* (TB). Especially relevant pathogens in the context of this invention for animals (such as livestock) may be a virus selected from the group comprising (the virus causing) Newcastle disease (chicken), Avian influenza (chicken), Swine influenza (pigs), Porcine reproductive and respiratory syndrome virus (PRRSV) (pigs), (the virus causing) Foot and Mouth Disease (FMD) (cattle), but also bacteria, such as the bacterium causing Bovine respiratory disease (cattle). However, the virus may also be another type of virus, like the Ebola virus. Especially, the virus may be a virus that enters the host body via the respiratory track.

The term "attenuated pathogen" may herein especially refer to a weakened pathogen with (severely) limited infectivity and/or replication (or "reproduction") capabilities, while (at least partially) having an (essentially) natural display of antigens on a surface of the pathogen, i.e., the attenuated pathogen may on a surface level resemble the corresponding unattenuated (or "natural") pathogen, but may internally be damaged. Hence, the immune system of a human or animal may be trained based on the antigens presented on the surface of the attenuated pathogen to recognize the (unattenuated) pathogen, thereby effectively vaccinating the human or animal. In particular, the attenuated pathogen may have sustained (substantial) damage to its genetic material, especially to its polynucleotides, such as DNA, or such as RNA, which may severely hamper the ability of the pathogen to successfully replicate in a host organism.

Hence, the invention may provide a method to vaccinate a human and/or animal using attenuated pathogens, which, due to substantial damage to their genetic material, may (essentially) be unable to replicate in the human or animal.

In embodiments, the indoor space may especially be a room in a building, especially a building configured for hosting humans, such as in a house, an office, a community center, a mall, or a hospital.

In further embodiments, the indoor space may especially be a room in a farm building, especially a farm building configured for hosting animals, such as a milking parlour, a barn, and a shelter. In such embodiments, the room may be open at one or more sides, such as may be common in farm building exposed to mild or hot climates. Hence, the term "indoor space" does not necessarily refer to a sealed off space, but may, in embodiments, be (partially) open to outside air.

In further embodiments, the indoor space may be functionally coupled with a climate control system, i.e., a climate control system is configured to control one or more environmental parameters in the indoor space.

In embodiments, the animal may especially be a farm animal, such as a farm animal selected from the group comprising chicken, pigs, cows, sheep, horses, goats, donkeys, llama, alpaca, rabbits, guinea pigs, and camels, especially from the group comprising pigs, chicken, cows, sheep, goats, and horses. In particular, the invention may be especially relevant for animals typically kept in large groups, i.e., in intensive animal farming.

The system, especially the control system (see below) may have an operational mode, especially a treatment mode. The term "operational mode" may also be indicated as "controlling mode". The system, or apparatus, or device (see further also below) may execute an action in a "mode" or "operational mode" or "mode of operation". Likewise, in a method an action, stage, or step may be executed in a "mode" or "operation mode" or "mode of operation". This does not exclude that the system, or apparatus, or device may also be adapted for providing another operational mode, or a plurality of other operational modes. Likewise, this does not exclude that before executing the mode and/or after executing the mode one or more other modes may be executed. However, in embodiments a control system (see further also below) may be available, that is adapted to provide at least the operational mode. Would other modes be available, the choice of such modes may especially be executed via a user interface, though other options, like executing a mode in dependence of a sensor signal or a (time) scheme, may also be possible. The operational mode may in embodiments also refer to a system, or apparatus, or device, that can only operate in a single operation mode (i.e. "on", without further tunability).

In embodiments, in the treatment mode, the system may be configured to subject first air comprising an airborne pathogen to first radiation, i.e., the system may be configured to provide first radiation to first air comprising an airborne pathogen.

The first radiation may especially comprise a spectral power distribution selected to attenuate the airborne pathogen, i.e., by exposing the airborne pathogen to the first radiation, an attenuated airborne pathogen may be provided.

In particular, polynucleotides and (surface) proteins, such as antigens, may be specifically targeted by selecting wavelengths that are differentially absorbed by the respective molecule. For instance, the first radiation may comprise one or more wavelengths that result in dimerization of polynucleotides, thereby damaging, especially (effectively) destroying, the genetic material of the pathogen, while the one or more wavelengths are (essentially) not absorbed by other molecules in the cell, such as proteins and lipids.

In embodiments, the one or more wavelengths may especially be selected based on the pathogen, such as based on a pathogen type, based on a pathogen presence, and based on environmental conditions. In particular, based on the understanding of the pathogen (its molecular composition), the pathogen load in the air, and the environmental conditions the pathogen resides in/is embedded in, the wavelength type(s), doses (energy x time) and wavelength setting ratios (e.g., a ratio of 260 and 220 nm light) may be selected to provide a full deactivation of the polynucleotide (RNA or DNA) combined with a minimal/limited damage to the remaining immune-inducing parts of the pathogen ('dead' virus components).

In particular, in general, and as described in the table below, wavelengths in the range of about 100 - 420 nm may be used to disinfect spaces from pathogens, indiscriminate towards the exact mechanism of inactivation. For instance, different wavelengths of radiation may have different properties and thus may have different compatibility with human presence and may have different effects when used for disinfection (Table 1).

**Table 1: Properties of different types of disinfection radiation**

| Name | Short name | Wavelength (nm) | (Relative) sterilization effectiveness | | Safe Radiatio n | Vitamin D generatio n | Ozone generatio n |
|---|---|---|---|---|---|---|---|
| | | | Bacteri a | Viruses | | | |
| Violet | V | 380-420 | +/- | - | + | | |
| Ultraviolet A | UV-A | 315-380 | + | - | + | | |
| Ultraviolet B | UV-B | 280-315 | + | +/- | +/- | + | |
| Near ultraviolet C | Near UV-C | 230-280 | + | + | - | | |
| Far ultraviolet | Far UV | 190-230 | + | + | + | | +/- |
| Extreme ultraviolet C | Extrem e UV-C | 100-190 | + | + | - | | + |

Each UV type / wavelength range may generally have different benefits and/or drawbacks. Relevant aspects may be (relative) sterilization effectiveness, safety (regarding radiation), vitamin D production (in a skin of a human being or animal), and ozone production (as result of its radiation). Depending on an application a specific type of UV light or a specific combination of UV light types may be selected and provides superior performance over other types of UV light. UV-A may be (relatively) safe and may inactivate, especially kill, bacteria, but may be less effective in inactivating (especially killing) viruses. UV-B may be (relatively) safe when a low dose (i.e. low exposure time and/or low intensity) is used, may kill bacteria, and may be moderately effective in killing viruses. UV-B may also have the additional benefit that it can be used effectively in the production of vitamin D in a skin of a person or animal. Near UV-C may be relatively unsafe, but may effectively kill bacteria and viruses. Far UV (or far UV-C) may also be effective in killing bacteria and viruses, but may be (relatively to other UV-C wavelength ranges) (rather) safe. Far-UV light may generate some ozone which may be harmful for human beings and animals. Extreme UV-C may also be effective in killing bacteria and viruses, but may be relatively unsafe. Extreme UV-C may generate ozone which may be undesired when exposed to human beings or animals. In some application ozone may be desired and may contribute to disinfection, but then its shielding from humans and animals may be desired. Hence, in the table "+" for ozone production especially implies that ozone is produced which may be useful for disinfection applications, but may be harmful for humans / animals when they are exposed to it. Hence, in many applications this "+" may actually be undesired while in others, it may be desired.

Typically, UVC disinfection/prevention of disease transmission efforts may be indiscriminate to how the deactivation is achieved (RNA-DNA/genetic damage and/or protein/recognition damage). Hence, for e.g. the use of far UVC (e.g., 222 nm) a same or even higher infectivity reduction might be found as for the longer wavelengths (such as 254 nm, or higher), but that the mechanism might differ. In particular, far UVC may typically cause mainly/more protein damage, whereas higher wavelengths, such as wavelengths ≥ 254 nm especially ≥ 255 nm, such as ≥ 260 nm, may cause mainly/more (relatively) nucleotide dimerization. However, the absorption spectra may differ between different pathogens.

For instance, Malayeri et al., "Fluence (UV Dose) Required to Achieve Incremental Log Inactivation of Bacteria, Protozoa, Viruses and Algae", International Ultraviolet Association Inc News 18(3):4-6 and supplemental tables, 2016, which is herein incorporated by reference, describes the response of various microorganisms to ultraviolet light. In particular, it describes the Fluence in mJ/cm² resulting in different log reductions for different microorganisms, also in view of different UV sources. For instance, certain pathogens might have specific envelopes of proteins that may largely block the often used 254 nm dimerization wavelength. In such cases, one might select a longer wavelength (e.g., 280 - 290 nm) that may have a relatively enhanced specificity for RNA/DNA over the (envelop) proteins, despite a efficiency of deactivating RNA/DNA compared to 254 nm.

In general, wavelengths selected from the group comprising 222 nm, 254 nm, and 275 nm may be particularly suitable for causing dimerization of the DNA and/or RNA of the pathogen. However, wavelengths of 222 nm may further cause (substantial) damage to proteins. Hence, wavelengths of (about) 254 nm or (about) 275 nm may be particularly suitable to target polynucleotides, especially resulting in dimerization thereof, while largely leaving proteins intact. Hence, in embodiments, the first radiation may comprise a wavelength selected from a first wavelength range comprising the range of 100 - 380 nm, especially from the range of 100 - 300 nm, such as at least comprising 254 nm, or such as at least comprising 275 nm. In particular, the one or more wavelengths may be selected to balance efficiency at causing dimerization of polynucleotides and being (essentially) inert with respect to (damaging) proteins. Hence, in embodiments, the first radiation may comprise a wavelength selected from a first wavelength range comprising the range of 240 - 300 nm, especially from the range of 250 - 300 nm, such as from the range of 255 - 300 nm.

In further embodiments, at least 50%, such as at least 70%, especially at least 90%, such as at least 95%, including (essentially) 100%, of the spectral power of the first radiation may be in the first wavelength range.

For instance, as demonstrated by the effective mRNA COVID-19 vaccines that make use of in-cell fabrication of S-proteins as immune trigger, it may, for COVID-19, be highly preferred that the S-proteins of the virus are (essentially) not modified by the first radiation, as the intact S-proteins may serve as recognition elements for host cell attachment and host cell entry. Therefore, for COVID-19, and potentially for other corona viruses, the first radiation may be selected for the above purpose (ratio of light absorption of RNA macromolecule versus protein macromolecule). In particular, in such embodiments, 254 nm may, for instance, be preferable over 222 nm light.

Further, the composition of the droplets/aerosolized particles, also referred to as airborne particles, can influence the selection of the first radiation. Expelled particles from an infectious person (sputum, phlegm) may be formed from mucus, which may be a complex aqueous fluid that owes its viscoelastic, lubricating and hydration properties to the glycoprotein mucin combined with electrolytes, lipids and other smaller proteins. Such mucus particles may therefore show a larger absorption at the shorter wavelengths, such as 222 nm, versus 254 nm. Because of co-absorption of light by the mucin, a higher dose may be required to dimerize the RNA/DNA to attenuate the pathogen. Further, the proteins in the aerosol particles might also be substantially absorbing at around 254 nm, and therefore one might select a wavelength that is rather in the range of 270 - 290 nm, such as about 280 nm, to increase specificity for the RNA/DNA damaging effect relative to the proteins damaging effect.

Hence, the selection of a spectral power distribution, may be made based on one or more of the pathogen (type), a pathogen abundance, and environmental conditions, which may change over time. Hence, in embodiments, a spectral power distribution of the first radiation may be controllable in at least part of a wavelength range of 100-380 nm, especially in at least part of a wavelength range of 200 - 340 nm, such as in at least part of a wavelength range of 240 - 300 nm.

In further embodiments, the system comprises a radiation source. The radiation source may be configured to provide first radiation, especially to provide first radiation to the first air. In particular, in further embodiments, the spectral power distribution of the first radiation provided by the radiation source may be controllable, i.e., the radiation source may be controlled to select a spectral power distribution of the first radiation, especially in at least part of a wavelength range of 100-380 nm, especially in at least part of a wavelength range of 200 - 340 nm, such as in at least part of a wavelength range of 240 - 300 nm.

Hence, in the treatment mode, the system, especially the radiation source, may (be configured to) provide the first radiation to the first air comprising the airborne pathogen, especially to provide second air comprising an attenuated airborne pathogen.

In further embodiments, the system may comprise a control system. The control system may especially be configured to control the system, such as the radiation source.

The term "controlling" and similar terms herein may especially refer at least to determining the behavior or supervising the running of an element. Hence, herein "controlling" and similar terms may e.g. refer to imposing behavior to the element (determining the behavior or supervising the running of an element), etc., such as e.g. measuring, displaying, actuating, opening, shifting, changing temperature, etc.. Beyond that, the term "controlling" and similar terms may additionally include monitoring. Hence, the term "controlling" and similar terms may include imposing behavior on an element and also imposing behavior on an element and monitoring the element. The controlling of the element can be done with a control system. The control system and the element may thus at least temporarily, or permanently, functionally be coupled. The element may comprise the control system. In embodiments, the control system and the element may not be physically coupled. Control can be done via wired and/or wireless control. The term "control system" may also refer to a plurality of different control systems, which especially are functionally coupled, and of which e.g. one control system may be a control system and one or more others may be slave control systems.

Hence, in embodiments, the control system may be configured to control the radiation source. In particular, the control system may be configured to control the spectral power distribution of the first radiation, such as in the treatment mode, especially in dependence of the airborne pathogen to be attenuated.

In embodiments, the phrase "in dependence of the airborne pathogen to be attenuated", and similar phrases, may especially indicate in dependence of the type of airborne pathogen to be attenuated. Further, in embodiments it may indicate in dependence of a concentration (in the space) of the airborne pathogen to be attenuated.

As indicated above, the first radiation may especially be selected (or controlled) to specifically damage genetic material of the pathogen. In embodiments, the pathogen may comprise a core part comprising genetic material and an outer part comprising outer part material, especially wherein the outer part material comprises one or more of proteins, lipids and polysaccharides. In further embodiments, a spectral power distribution of the first radiation may be selected such that in a part of the wavelength range of the first radiation, such as in the wavelength range of 100 - 380 nm, a ratio between absorption of radiation by the genetic material and absorption of radiation by the outer part material, especially proteins, in that part of the wavelength range is more than 1, such as at least 2, such as at least 4, especially at least 8.

In further embodiments, over the (total) wavelength range of the first radiation, the ratio between absorption of radiation by the genetic material and absorption of radiation by the outer part material in that part of the wavelength range is more than 1, such as at least 2, such as at least 4, especially at least 8.

The ratio between absorption of radiation by the genetic material and absorption of radiation by the outer part material, especially proteins, may have a (pathogen-specific) highest value Rₘₐₓ at a specific wavelength in the wavelength range of the first radiation. In embodiments, the spectral power distribution may be selected such that the ratio between absorption of radiation by the genetic material and absorption of radiation by the outer part material, especially proteins, is at least 0.7*Rₘₐₓ, such as at least 0.8*Rₘₐₓ, especially at least 0.9*Rₘₐₓ, including Rₘₐₓ.

In particular, wavelengths in the range of 255 - 300 nm, especially in the range of 260 - 300 nm, may have beneficial properties regarding the selective damaging of genetic material compared to the damaging of proteins.

Hence, in embodiments, in an operational mode of the system, especially in the treatment mode, the first radiation may have a first spectral power distribution of which at least 70% of the spectral power in the 100-300 nm wavelength range is in the 255-300 nm wavelength range, especially at least 90%, such as at least 95%. Hence, the system, especially the radiation source, may be configured to provide first radiation (to first air), wherein the first radiation has a first spectral power distribution of which at least 70% of the spectral power in the 100-300 nm wavelength range is in the 255-300 nm wavelength range, especially at least 90%, such as at least 95%.

In particular, in further embodiments, in an operational mode of the system, especially in the treatment mode, at least 90%, such as at least 95%, of the spectral power of the first radiation in the 100-300 nm wavelength range may be in a focus wavelength range. In embodiments, the focus wavelength range may especially have a lower wavelength limit of at least 250 nm, such as at least 255 nm, especially at least 260 nm. In further embodiments, the focus wavelength range may have a lower wavelength limit of at least 270 nm, such as at least 280 nm, especially at least 290 nm. In further embodiments, the focus wavelength range may have an upper wavelength limit of at most 300 nm, such as at most 290 nm, especially at most 280 nm. In further embodiments, the focus wavelength range may have an upper wavelength limit of at most 270 nm, such as at most 260 nm.

In further embodiments, the system may comprise a second radiation source configured to provide second radiation, wherein in an operational mode of the system the second radiation has a second spectral power distribution of which at least 90% of the spectral power in the 100-300 nm wavelength range is in a second focus wavelength range, especially wherein the second wavelength range comprises the range of 100-255 nm. In particular, it may be advantageous to have two (or more) radiation sources that may be separately controlled to provide radiation for attenuating (or killing) the airborne pathogen, such as separately controlled in view of one or more of pathogen type and/or pathogen abundance.

In further embodiments, at least 50%, such as at least 70%, especially at least 90%, such as at least 95%, including (essentially) 100%, of the spectral power of the second radiation in the range of 100 - 300 nm may be in the second focus wavelength range.

Hence, in further embodiments, the system may comprise a plurality of radiation sources.

Practically, the first air may comprise a plurality of (same) pathogens, wherein the first radiation differentially affects different individual pathogens, akin to soap that kills 99% of bacteria. Hence, depending on a spectral power distribution and an intensity of the first radiation, a percentage of pathogens may be attenuated. With a low percentage of attenuation, a human or animal in the indoor space may be exposed to (too) high levels of a live pathogen. However, with a high percentage of attenuation, the surface of the pathogen may be (detrimentally) affected by the first radiation, despite a selection of the spectral power distribution that predominantly targets polynucleotides. Hence, in embodiments, a spectral power distribution of the first radiation and/or an intensity of the first radiation may be selected to provide at least a 1-log reduction of the airborne pathogen, i.e., that 90% of the airborne pathogen in the first air is attenuated (or killed). In further embodiments, a spectral power distribution of the first radiation and/or an intensity of the first radiation may be selected to provide at least a 2-log reduction of the airborne pathogen, especially at least a 3-log reduction, such as at least a 4-log reduction. In further embodiments, the spectral power distribution of the first radiation and/or the intensity of the first radiation may be selected to provide at least a 5-log reduction of the airborne pathogen, especially at least a 6-log reduction. In further embodiments, a spectral power distribution of the first radiation and/or an intensity of the first radiation may be selected to provide at most a 8-log reduction of the airborne pathogen, especially at most a 7-log reduction, such as at most a 6-log reduction. In further embodiments, a spectral power distribution of the first radiation and/or an intensity of the first radiation may be selected to provide at most a 5-log reduction of the airborne pathogen, especially at most a 4-log reduction, such as at most a 3-log reduction.

It will be clear to the person skilled in the art that the efficiency of the attenuation of the pathogen may depend both on the irradiance and the spectral power distribution of the first radiation. For instance, some first wavelengths may be more prone to lead to dimerization of DNA or RNA than others. Further, a microorganism, such as a bacterium, may have DNA-repair mechanisms and could therefore cope with DNA dimerization up to a certain point. The term "attenuated" herein refers to the first radiation and the first irradiance being selected such that the microorganisms are effectively inactivated (or "killed"), especially such that the pathogens are modified in such a way that they cannot further infect and/or reproduce. It will further be clear to the person skilled in the art that this may depend on a duration of the exposure to the first radiation, and the person skilled in the art will select suitable wavelengths and irradiance in view of the intended application.

In further embodiments, the system, especially the radiation source, may be configured to (during the treatment mode) provide the first radiation with a first irradiance selected from the range of 0.5 - 200 mW/cm², especially from the range of 1 - 100 mW/cm², such as from the range of 2 - 80 mW/cm². In particular, the first irradiance may be (defined at) at a distance from the radiation source selected from the range of 0.05 - 2 m, such as from the range of 0.1-1 m, especially from the rage of 0.2 - 0.8 m.

The present invention may further take advantage of a measurable output when microorganisms are exposed to (germicidal) UV radiation. In particular, the absorption of UV energy by a microorganism may result in the formation of new bonds between adjacent nucleotides, creating double bonds or dimers. Dimerization of adjacent nucleotides in DNA or RNA, particularly involving thymine, may be the most common photochemical damage as initiated by the germicidal effect of far UVC (e.g. 254 nm) and may lead to attenuation of microorganisms. Formation of dimers in the RNA / DNA of bacteria and viruses may prevent replication and may (essentially) result in an inability to infect. As an effect of the dimerization, radiation may be emitted, e.g., with a peak at about 600nm. This radiation can be detected, which makes it possible to detect the attenuation reaction and the attenuated microorganism, especially the attenuated pathogen. Hence, by providing UV radiation to microorganisms, the microorganisms may be attenuated, which provides a measurable output, which may be indicative of the presence of the microorganisms as such, of a concentration or a number of present microorganisms in a space or on a surface, as well as of their attenuation. Thereby, the system of the invention may facilitate both the attenuating and the monitoring of the pathogen(s).

Hence, exposing the airborne pathogen to the first radiation may cause dimerization of polynucleotides in the airborne pathogen, corresponding to attenuation of the airborne pathogen, which may further cause the emission of third radiation. The third radiation may be both indicative of a pathogen concentration in the first air, and of (a degree of) pathogen attenuation.

Hence, in embodiments, the system may comprise a radiation sensor. The radiation sensor may be configured to detect third radiation (originating from the attenuated airborne pathogen) having a third wavelength in the range of 550-680 nm. In further embodiments, the radiation sensor may be configured to detect the third radiation and to provide a related third sensor signal to the control system.

The term "X related signal" may herein refer to a signal that is related to the detected X. In particular, the X-related signal may comprise raw and/or processed data related to the (detected) X. Hence, for example, the term "related third sensor signal" may herein refer to a signal that is related to the detected third radiation. In particular, the related sensor signal may comprise raw and/or processed data related to the (detected) third radiation. In embodiments, the related third sensor signal may comprise (raw and/or processed) data related to a spectral power at the third wavelength or a spectral power distribution of at a plurality of third wavelengths in a third wavelength range. In further embodiments, the related sensor signal may comprise temporal data, such as time stamps, related to the spectral powers. Hence, the related sensor signal may comprise data related to changes over time in the spectral power at a third wavelength, especially at a plurality of third wavelengths.

The third wavelength of the third radiation, especially the distribution of third wavelengths of the third radiation, may vary between different microorganisms. For instance, RNA viruses may be devoid of DNA, and may therefore be devoid of the nucleobase thymine. Similarly, most DNA viruses may be devoid of RNA, and may therefore be devoid of the nucleobase uracil. However, other microorganisms, including bacteria, archaea and fungi, generally have both DNA and RNA, and will thus comprise both thymine and uracil, but may differ in the relative abundances thereof. Hence, the third wavelength, especially the distribution of third wavelengths, may thus be indicative of the type of microorganisms, such as a ratio between third wavelengths corresponding to DNA and third wavelengths corresponding to RNA being indicative of the type of microorganisms. Further, the prevalence of different nucleobases may also vary between, for instance, two DNA viruses, or between two bacteria. Hence, the third wavelength, especially the distribution of third wavelengths, may thus also contribute to identification of the microorganisms.

The radiation sensor system may especially comprise a detector configured to detect the third radiation. In embodiments, the detector may be configured to detect the third radiation. The detector may especially comprise a spectrometer.

The radiation sensor system may especially comprise one or more optical components, such as one or more optical components selected from the group comprising an optical filter, a lens, a polarizer, a beam splitter, a mirror, and a retroreflector. In particular, the radiation sensor system may comprise an optical filter configured to selectively transmit the third radiation, such as to selectively transmit third radiation having the third wavelength range, especially wherein the optical filter is arranged between (at least part of) the enclosure and a detector of the radiation sensor system. In further embodiments, the radiation sensor system may comprise a third optical filter configured to selectively block the third wavelength, such as to selectively block radiation in the third wavelength range, especially wherein the third optical filter is arranged downstream of the enclosure with respect to the detector of the radiation sensor system. Hence, the third optical filter may be arranged to reduce ambient radiation in the third wavelength range.

The third radiation emitted by the microorganisms may be polarized. Hence, in embodiments, the radiation sensor system may comprise a polarizer. Thereby, a higher signal/noise ratio may be obtained.

In particular, the system, especially the treatment mode of the system, is not restricted to the presence of the pathogen. Hence, during the treatment stage, the radiation sensor system may detect in the third wavelength range, but may not detect any third radiation. Hence, the (related) third sensor signal may also be indicative of the absence of detected third radiation.

In further embodiments, the system, especially the control system, may be configured to control (in an operational mode) the treatment mode in dependence of the third radiation. In particular, the system, especially the control system, may be configured to control the radiation source in dependence of the third radiation, especially based on the related third sensor signal.

The phrase "to control the treatment mode" and similar phrases may herein especially refer to controlling one or more aspects of the treatment mode, such as controlling a spectral power distribution and/or intensity of the first radiation during the treatment mode, but also, for instance, controlling an exposure time of the first air to the first radiation.

In embodiments, the system may further comprise a pathogen sensor. The pathogen sensor may be configured to sense the airborne pathogen in the indoor space and to provide a related pathogen sensor signal. In particular, the pathogen sensor may be configured to sense (live) airborne pathogen in the indoor space and to provide a related pathogen sensor signal, especially to the control system. In such embodiments, the system, especially the control system, may be configured to control in an operational mode the treatment mode in dependence of the pathogen sensor signal. Hence, if a concentration of (live) airborne pathogen exceeds a (predefined) threshold, a spectral power distribution and/or an intensity of the first radiation may be adjusted to provide a larger log reduction, especially attenuation, of the airborne pathogen.

As indicated above, the environment may influence the effect of the first radiation on the airborne pathogen. Hence, the system further comprises an indoor climate sensor configured to sense an indoor climate parameter in the indoor space and to provide a related indoor climate sensor signal, especially to the control system. The system, especially the control system, is configured to control in an operational mode the treatment mode in dependence of the indoor climate sensor signal by controlling one or more of (i) a spectral power distribution of the first radiation, (ii) an intensity of the first radiation and an exposure time of the first air to the first radiation.

The indoor climate parameter may be selected from the group comprising temperature, humidity, airflow, airborne particle size, airborne particle size distribution and air composition. The air composition may also relate to the composition of the airborne particles.

Typically, airborne particles are present in the indoor space, which may comprise pathogens. Particularly, the airborne particle size and/or airborne particle size distribution, and possibly the airborne particle (chemical) composition, may influence the selection of the first radiation. The size, or size distribution, or (chemical) composition of the airborne particles may be influenced by parameters like temperature, humidity, airflow and air composition of the indoor space. Airborne with a larger size or a larger particle size distribution may require a higher intensity of first radiation and/or a different spectral power distribution of first radiation in order to attenuate airborne pathogens present in the airborne particles or in the air itself, due to absorption of the first radiation by the airborne particles. By doing so the effectiveness of the attenuation of the airborne pathogens is improved.

In embodiments, the control system may be configured to further receive and/or retrieve input from one or more of an input signal of a user interface, a sensor signal, a timer, and an external database.

Hence, in embodiments, the control system may comprise a user interface, which may be used to provide input for the control system, or especially to provide instructions to the (control) system.

In further embodiments, the system, especially the control system, may be configured to retrieve input, especially information (or "data"), from an external database. In embodiments, the information (or data) may comprise details of the molecular structure of pathogens, especially of the airborne pathogen, and more especially of its light absorption characteristics for the various elements (parts) of the pathogen, especially of different molecules in the pathogen. In further embodiments, the system, especially the control system, may be configured to control the treatment mode in dependence on the information from the external database. For instance, an external database may comprise information on recent outbreaks of different pathogens that may be targeted by the system of the invention. Hence, the system may be configured to (semi-)automatically adjust its operational parameters in view of recent disease outbreaks. For example, an external database might be related to an ongoing pandemic; so one would know the type of pathogen (e.g., CoV2, flu,...) and could take the required settings of the apparatus (wavelength, dose) from a library/table with settings from known pathogens/diseases. The external database may further comprise information on molecular analysis/molecular composition of a specific pathogen and the absorption characteristics by the various parts of the pathogen (such as genetic material and proteins).

In further embodiments, the first radiation may have a controllable spectral power distribution within at least part of a wavelength range of 100-380 nm, especially in at least part of a wavelength range of 255-300 nm, wherein the control system is configured to control the treatment mode in dependence of one or more of an input signal of a user interface, a sensor signal, a timer, and external database information, especially to control one or more of the spectral power distribution of the first radiation, the intensity of the first radiation, and/or an exposure time of first air to the first radiation.

In a second aspect, the invention may further provide an arrangement comprising (a) the system of the invention and (b) an indoor space. Especially, the system may be configured to (a) subject in the treatment mode first air comprising the airborne pathogen to first radiation to attenuate the airborne pathogen to provide second air with the attenuated airborne pathogen, and to (b) introduce the second air with the attenuated airborne pathogen in the indoor space.

In embodiments, the arrangement may comprise a single space comprising the indoor space. For instance, a pathogen source may be in the indoor space, and the system, especially the radiation source, may be configured to provide the first radiation to the first air in the indoor space, thereby providing second air in the indoor space. An advantage of such embodiment may be that a relatively large exposure cross section may be available, such as the ceiling and/or (parts of) the walls of the indoor space. In further embodiments, the radiation source may comprise an upper air luminaire configured to provide the first radiation to the ceiling and/or an (upper) part of a wall. Thereby, exposure of a human and/or an animal in the indoor space to the first radiation may be limited, especially eliminated.

In embodiments, the arrangement may comprise a tent, such as a hospital tent (or "medical tent").

However, the arrangement may also comprise a plurality of (separated) spaces, such as rooms in a hospital, i.e., in embodiments the arrangement may comprise (a part of) a building, such as a hospital.

In further embodiments, the arrangement may comprise a second indoor space, especially wherein the second indoor space is configured to host a pathogen source, such as a patient, or such as a sick animal. In further embodiments, the system may be configured to retrieve the first air from the second indoor space. Hence, the arrangement may be configured to (have the system) (actively) retrieve first air comprising an airborne pathogen, to attenuate the airborne pathogen, and to provide second air comprising an attenuated airborne pathogen to the first space. Thereby, humans or animals in the first space may be exposed to an attenuated form of the airborne pathogen, which (natural) airborne pathogen infects patients/sick animals.

In particular, such arrangement may provide a decoupling between providing the attenuated pathogen for vaccination use afterwards, and providing the attenuated pathogen to a human and/or animal. Hence, this provides the option to proceed till 'ready' in the second indoor space or in the enclosure, optionally including a quality check, such as with sensor elements described herein, and then provide the second air with the attenuated pathogen to the indoor space.

In further embodiments, the second indoor space may be configured to host human patients, especially human patients having a respiratory disease.

In further embodiments, the second indoor space may be configured to host sick animals, especially sick animals having a respiratory disease.

In further embodiments, the first indoor space and the second indoor space are comprised by a hospital, a hospital tent, an elderly home, a teaching building (like a school or university), etc.

In embodiments, one or more of the first indoor space and the second indoor space may comprise the pathogen sensor. Especially, the first indoor space may comprise the pathogen sensor. Alternatively or additionally, the second indoor space may comprise the pathogen sensor.

In further embodiments, the system, especially the control system, may be configured to control in an operational mode the treatment mode in dependence of one or more of the pathogen sensor signal, and (ii) the external database information.

In embodiments, the system, especially the radiation source, may be configured to provide the first radiation to the indoor space, such as to a ceiling and/or part of a wall in the indoor space. Hence, the system may especially be an open system. In particular, an open system may have the benefit that a larger exposure cross section may be available, as compared to the inside of a device, as, for instance also UV radiation that 'shines along the ceiling or walls' of the indoor space may be used, obviously taking care that people do not get irradiated. For this, for example, upper air UV luminaires may be used, i.e., the radiation source may, in embodiments, comprise an upper air (UV) luminaire.

In embodiments, the system may comprise one or more enclosure walls. In further embodiments, the system, especially the one with the one or more enclosure walls, may (be configured to) define at least part of an enclosure. The term "enclosure" may herein especially refer to a space that is (at least partially) enclosed. Hence, also the term "enclosure space" may be used herein. In particular, the enclosure may be (essentially) shielded from external radiation, especially from external radiation in the third wavelength range. Hence, the enclosure may especially be an optical enclosure. In embodiments, the system may comprise one or more wall elements configured to define at least part of an enclosure, such as to at least partially enclose a space. For instance, the system may comprise one or more wall elements configured to be arranged against a surface, wherein the one or more wall elements define an enclosure together with the surface. In further embodiments, the one or more wall elements may define an enclosure (without a further surface). In particular, in embodiments, the system may comprise the enclosure.

In further embodiments, the treatment mode (or the method) may comprise providing the first air to the enclosure, providing the first radiation to the first air in the enclosure to provide the second air, and providing the second air (from the enclosure) to the indoor space.

Hence, in further embodiments, the enclosure may have one or more openings, especially at least two openings, such that a gas, may pass through the enclosure. In further embodiments, the enclosure may be a channel, such as a channel with a tube-shape, such as a tunnel. Hence, in embodiments, the one or more wall elements may define a channel, especially a channel with openings at opposite ends.

In further embodiments, the radiation sensor system may be configured to successively detect third radiation in or originating from different parts of the enclosure. In particular, the radiation sensor system may comprise a plurality of detectors configured to detect the third radiation (in or originating from different parts of the enclosure).

The enclosure may, in embodiments, be (configured) shielded from ambient light, especially from ambient light in the second wavelength range. The term "ambient light" may herein especially refer to light in the immediate surroundings of the enclosure, such as light incident on an external surface of one or more enclosure walls defining the enclosure. Hence, the enclosure walls may be configured to provide an enclosure shielded from ambient light.

In particular, by shielding the enclosure of ambient light, especially of ambient light in the second wavelength range, the system may have a higher sensitivity for detecting (the attenuation of) the airborne pathogen, especially due to a lower background "noise", i.e., due to a higher signal/noise ratio.

In embodiments, in the operational mode, the radiation sensor system and the control system may be configured to determine a radiative decay of the third radiation at the third wavelength as function of time. In such embodiments, the control system may (be configured to) determine a microorganism-related parameter based on the radiative decay. The microorganism-related parameter may, for instance, relate to the type of airborne pathogen.

In embodiments, especially during the treatment mode, the radiation source may be configured to (essentially) continuously provide the first radiation (to the enclosure). However, it may not be required to continuously provide the first radiation. Hence, in further embodiments, the system, especially the control system, may be configured to periodically execute the treatment mode.

With respect to the first irradiance of the first radiation, the first irradiance may especially be determined at a surface to which the first radiation is provided, such as a surface of an object, or such as an enclosure wall. Hence, in embodiments the radiation source may be configured to provide the first radiation to a section of an enclosure wall (of the one or more enclosure walls) with an irradiance selected from the range of 0.5 - 200 mW/cm², such as selected from the range of 1 - 100 mW/cm², especially from the range of 2 - 80 mW/cm². In particular, the section of the enclosure wall may be in a line of sight from the radiation source(s), especially the section of the enclosure wall may be arranged at an angle ≤ 5° from an optical axis of the radiation source. In embodiments, the section of the enclosure wall is at most 1 m from the radiation source, such as at most 0.5 m, especially at most 0.1 m. Hence, in an embodiment a reference distance for determining the first irradiance may be selected from the range of 0.1-1 m from the (respective) radiation source (especially determined along the optical axis). Hence, the radiation source may in embodiments be configured to provide the first radiation with a first irradiance selected from the range of 1 - 100 mW/cm² at a distance from the radiation source selected from the range of 0.1-1 m.

In embodiments, the system may comprise an air treatment system, especially wherein the enclosure, or one or more of the enclosure walls, comprises at least part of the air treatment system, such as (at least part of) a pipe. For instance, the system may be arranged such that one or more walls of a pipe of the air treatment system form the one or more enclosure walls. The air treatment system may especially comprise a HVAC system. Hence, in embodiments, the system may be configured to attenuate pathogens in ventilated air. In particular, the control system may be configured to control the treatment mode in dependence of one or more of an air flow, a temperature, a residence time in the air treatment system, and a humidity. For instance, the control system may be configured to control an air flow, especially such that first air comprising a number of the pathogen above a pre-defined threshold is routed away from rooms in which people reside, or, rather, that second air comprising the attenuated pathogen is routed towards the indoor space.

In embodiments, the air treatment system may comprise a heating, ventilation, and air conditioning (HVAC) system.

In embodiments, the method may especially be executed using the system of the invention.

In a further aspect, the invention may provide a method for distributing an attenuated airborne pathogen in an indoor space for humans or animals. The method may comprise: (a) subjecting in a treatment mode first air comprising an airborne pathogen to first radiation to attenuate the airborne pathogen to provide second air with the attenuated airborne pathogen. The method may further comprise: (b) introducing the second air with the attenuated airborne pathogen in the indoor space.

The method may especially be executed using the system of the invention.

The method may especially comprise executing the treatment mode of the system of the invention.

In a further aspect, the invention may provide a computer program product comprising instructions for execution on a control system functionally coupled to a system, wherein the instructions, when executed by the control system, cause the system to carry out the method according of the invention or the treatment mode of the (system of the) invention.

In a further aspect, the invention may provide a data carrier, carrying thereupon program instructions which, when executed by a control system functionally coupled to a system, cause the system to carry out the method of the invention or the treatment mode of the (system of the) invention.

In a specific embodiment, the radiation source may comprise a light source, especially a solid state LED light source (such as a LED or laser diode), or especially a mercury lamp, or especially an excimer light source. The term "light source" may also relate to a plurality of light sources, such as 2-20 (solid state) LED light sources. Hence, the term LED may also refer to a plurality of LEDs.

The embodiments described herein are not limited to a single aspect of the invention. For example, an embodiment describing the method may, for example, further relate to the system, especially to an operational mode of the system, such as to the treatment mode of the system, or especially to the control system. Similarly, an embodiment of the system describing an operation of the system may further relate to embodiments of the method. In particular, an embodiment of the method describing an operation (of the system) may indicate that the system may, in embodiments, be configured for and/or be suitable for the operation. Similarly, an embodiment of the operational mode, especially the treatment mode, of the system may indicate that the method may comprise executing the indicated steps.

### BRIEF DESCRIPTION OF THE DRAWINGS

Embodiments of the invention will now be described, by way of example only, with reference to the accompanying schematic drawings in which corresponding reference symbols indicate corresponding parts, and in which:

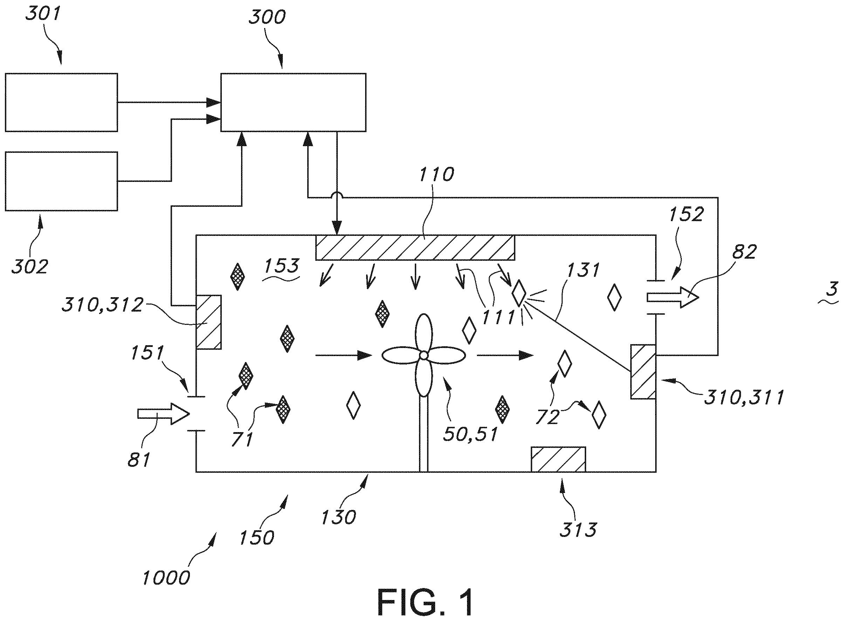

Fig. 1-3 schematically depict embodiments of the system of the invention.

The schematic drawings are not necessarily on scale.

### DETAILED DESCRIPTION OF THE EMBODIMENTS

Fig. 1 schematically depicts an embodiment of the system 1000 for distributing an attenuated airborne pathogen 72 in an indoor space 3 for humans 10 or animals 20. In the depicted embodiment, the system 1000 may be configured to subject in a treatment mode first air 81 comprising an airborne pathogen 71 to first radiation 111 to attenuate the airborne pathogen 71. Thereby, the system 1000 may provide second air 82 with the attenuated airborne pathogen 72. In the treatment mode, the system 1000 may further be configured to introduce the second air 82, comprising the attenuated airborne pathogen 72, in the indoor space 3.

In embodiments, a spectral power distribution of the first radiation 111 may be controllable in at least part of a wavelength range of 100-380 nm, such as in the wavelength range of 200 - 300 nm, especially in the range of 255 - 300 nm. In particular, the system 1000 may comprise a control system 300 configured to control the spectral power distribution of the first radiation 111, especially in the treatment mode, and especially in dependence of the airborne pathogen 71 to be attenuated. Hence, the control system 300 may select an appropriate spectral power distribution in view of the airborne pathogen, such as in view of absorption spectra of polynucleotides and proteins of the airborne pathogen 71.

In the depicted embodiment, the arrows between the sensors 310, the user input 301, the external database 302, the control system 300, and the radiation source 110, may represent a flow of information, such as related sensor signals, external database information, and control signals.

Further, in the depicted embodiment, the system 1000 may comprise an airflow device 50, such as a fan 51, configured for drawing in the first air 81 and for providing the second air 82 to the indoor space 3. In particular, in the depicted embodiment, the system 1000 comprises an enclosure 130, wherein the airflow device 50 is configured to provide first air 81 to the enclosure, wherein the system is configured to provide the first radiation 111 to first air 81 comprising the airborne pathogen 71 in the enclosure 130, thereby providing second air 82 comprising the attenuated airborne pathogen 72, and wherein the airflow device 50 is configured for providing the second air 82 to the indoor space 3.

In the depicted embodiment, the system 1000 further comprises a radiation source 110 configured to generate the first radiation 111. In particular, in the treatment mode, the radiation source may (be configured to) provide the first radiation 111 to the first air 81.

In embodiments, the first radiation may comprise one or more wavelengths selected from a first wavelength range comprising the range of 100-300 nm, especially the range of 200 - 300 nm, such as the range of 250 - 300 nm.

The system, especially the control system, may further have an operational mode.

In embodiments, in the operational mode, especially in the treatment mode, of the system 1000, the first radiation 111 may have a first spectral power distribution of which at least 90% of the spectral power in the 100-300 nm wavelength range is in the 255-300 nm wavelength range. Radiation in this wavelength range may generally primarily damage genetic material, such as polynucleotides, of the airborne pathogen, while causing limited damage to proteins (and other macromolecules) of the airborne pathogen, which may allow an outer surface of the airborne pathogen to remain (relatively) intact, while attenuating the pathogen by virtue of the damage to its genetic material.

In embodiments, the control system 300 may be configured to control the treatment mode, especially via controlling the first radiation 111, such as by controlling the radiation source 110. In further embodiments, the control system may be configured to control a spectral power distribution of the first radiation 111. In further embodiments, the control system may be configured to control an intensity (or irradiance) of the first radiation 111. In further embodiments, the control system may be configured to control an exposure time of the first air 81 to the first radiation 111, such as by controlling the airflow device 50.

Hence, in embodiments, a spectral power distribution of the first radiation 111 and an intensity of the first radiation 111 may be selected to provide at least a 1-log reduction of the airborne pathogen 71. In particular, the control system may be configured to, in an operational mode, select a spectral power distribution of the first radiation 111 and an intensity of the first radiation to provide at least a 1-log reduction of (live) airborne pathogen 71, such as at least a 2-log reduction of the airborne pathogen 71, especially at least a 3-log reduction of the airborne pathogen.

In particular, the phrase "a 1-log reduction of the airborne pathogen 71" may refer both to 90% of the airborne pathogen being attenuated or killed.

The control system may further be configured to select the spectral power distribution and/or the intensity of the first radiation 111 in view of an abundance of the airborne pathogen 71 and a type of the airborne pathogen 71. For instance, if the airborne pathogen 71 is highly abundant in the first air 81, the control system 300 may select the spectral power distribution and/or the intensity of the first radiation 111 to provide a large log reduction of the airborne pathogen 71 in view of safety.

In further embodiments, the system 1000, especially the radiation source 110, may be configured to provide the first radiation 111 with a first irradiance selected from the range of 1 - 100 mW/cm², such as from the range of 2 - 80 mW/cm². Especially, the control system 300 may be configured to control the radiation source 110 to provide the first radiation 111 with a first irradiance selected from the range of 1 - 100 mW/cm², such as from the range of 2 - 80 mW/cm².

The control system 300 may especially control the treatment mode, such as by controlling the radiation source, in dependence of one or more sensors 310. Hence, in embodiments, the system 1000 may comprise one or more sensors 310, such as a radiation sensor 311, a pathogen sensor 312, and an indoor climate sensor 313. The sensors 310 may be configured to sense a (respective) parameter and to provide a related sensor signal to the control system 300. Hence, the control system may especially control the treatment mode in dependence of the related sensor signal(s).

In further embodiments, the system 1000 may comprise a radiation sensor 311. The radiation sensor 311 may be configured to detect third radiation 131 (originating from an attenuated airborne pathogen 72) having a third wavelength in the range of 550-680 nm, and especially to provide a related third sensor signal to the control system. In particular, the third radiation 131 may be radiation emitted by the airborne pathogen 71 upon polynucleotide dimerization. Hence, the third radiation 131 may be indicative of attenuation of the airborne pathogen 71. In further embodiments, the system 1000, especially the control system 300, may in an operational mode (be configured to) control the treatment mode in dependence of the third radiation 131, especially in dependence of the related third sensor signal.

In further embodiments, the system 1000 may further comprise a pathogen sensor 312. The pathogen sensor 312 may especially be configured to sense the airborne pathogen 71, especially in the indoor space 3, or especially in the enclosure 130. In further embodiments, the pathogen sensor 312 may be configured to sense the airborne pathogen 71 and to provide a related pathogen sensor signal, especially to the control system 300. In further embodiments, the system 1000, especially the control system 300, may be configured to control in an operational mode the treatment mode in dependence of the pathogen sensor signal.

In particular, the pathogen sensor 312 may be configured to determine one or more of an airborne pathogen type or an airborne pathogen abundance, especially an airborne pathogen type, or especially an airborne pathogen abundance.

In further embodiments, the system 1000 may comprise an indoor climate sensor 313. The indoor climate sensor 313 may be configured to sense an indoor climate parameter, especially in the indoor space 3, or especially in the enclosure 130. The indoor climate parameter may especially be selected from the group comprising temperature, humidity, airflow, and air composition. The indoor climate sensor 313 may further be configured to provide a related indoor climate sensor signal, especially to the control system 300. In further embodiments, the system 1000, especially the control system 300, may in an operational mode (be configured to) control the treatment mode in dependence of the indoor climate sensor signal.

In further embodiments, the control system 300 may be configured to retrieve and/or receive an input signal from a user interface 301 and/or from an external database 302. For instance, a user may inform the control system (300) via the user interface 301 on the presence of an airborne pathogen type of an airborne pathogen 71 in the first air 81, and the external database 302 may comprise structural information regarding the airborne pathogen 71. Such information may facilitate the control system 300 in selecting an appropriate spectral power distribution of the first radiation 111 to provide the second air 82 comprising the attenuated airborne pathogen 72.

In further embodiments, the spectral power distribution of the first radiation 111 may be controllable within at least part of a (first) wavelength range of 100-380 nm, especially in at least part of a (first) wavelength range of 255-300 nm. In particular, in such embodiments, the control system may be configured to control the spectral power distribution of the first radiation in at least part of the (first) wavelength range of 100-380 nm, such as in at least part of the (first) wavelength range of 255-300 nm. In further embodiments, the control system 300 may be configured to control the spectral power distribution in dependence of one or more of an input signal of a user interface, a sensor signal, a timer, and external database information, especially in dependence on the user interface, or especially in dependence on the sensor signal, or especially in dependence on the timer, or especially in dependence on the external database information.

Specifically, Fig. 1 schematically depicts a system 1000, especially a device 150, containing an air inlet 151 and an air outlet 152, with air continuously flowing through the device 150. The air inlet 151 may receive first air 81 originating from a (potential, preferably selected for that purpose) pathogen-shedding person (or animal), whereas the air outlet 152 may provide (processed) second air 82 towards a human 10 (or animal 20) that may inhale the second air 82, with the purpose to induce a protective immunological reaction. The device 150 may comprise an air duct 153 having a sufficient length (pathway) as to allow exposing the first air 81 and its constituents to first radiation 111 having one or more selected first wavelengths. This first radiation may be provided by tunable UV (and alike) light sources, wherein the tuning may especially include power (intensity) adaptation and periodicity (time) adaptation, such as adaptation based on time of day (e.g., depending on use of the indoor space), or such as based on the presence of infected and healthy persons/animals. In particular, a load of the airborne pathogen 71 in the air may be modified by the first radiation 111 in the device 150, in such a way that the airborne pathogen 71 is deactivated in its replication power, but kept intact with respect to antigen molecular parts, i.e., such that the airborne pathogen 71 is attenuated. The system 1000, especially the control system 300, may further be configured to take into account the various pathogen and environmental parameters as to achieve suitable selective pathogen attenuation.

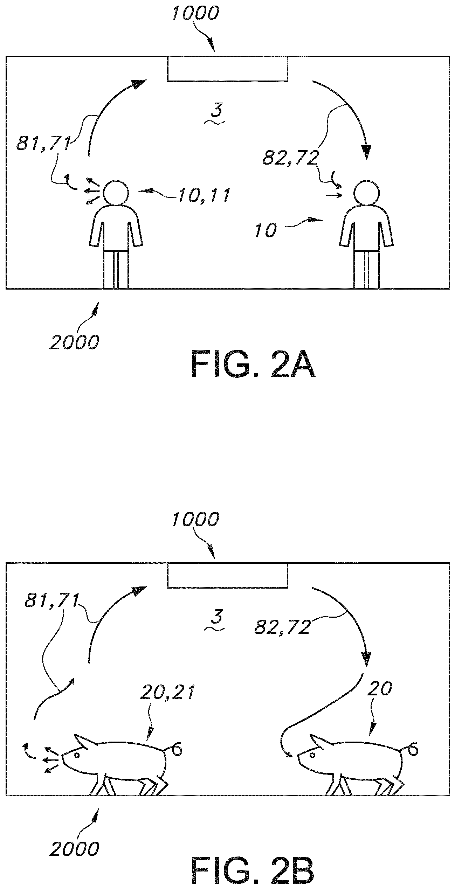

Fig. 2A-B schematically depict embodiments of an arrangement 2000 comprising the system 1000 and the indoor space 3. In the depicted embodiment, the system 1000 may be configured to subject in the treatment mode first air 81 comprising the airborne pathogen 71 to first radiation 111 to attenuate the airborne pathogen 71 to provide second air 82 with the attenuated airborne pathogen 72, and to introduce the second air 82 with the attenuated airborne pathogen 72 in the indoor space 3. In the depicted embodiment, the system 1000 may be configured to receive first air 81 comprising the airborne pathogen 71 from the indoor space 3, and to provide second air 82 comprise the attenuated airborne pathogen 72 to the indoor space 3.

Specifically, in the embodiment depicted in fig. 2A, the airborne pathogen 71 may be provided to (the first air 81 in) the indoor space 3 by a human patient 11, and the second air 82 comprising the attenuated airborne pathogen 72 may be provided to a (healthy) human 10 in the indoor space 3, which may trigger and train the immune system of the human 10, thereby providing resistance to subsequent exposure to the airborne pathogen 71.

Hence, in embodiments, the system may especially comprise an open system, which may provide access to a (relatively) large effective air processing volume/surface.

Fig. 2B schematically depicts a further embodiment, wherein the airborne pathogen 71 is provided to (the first air 81 in) the indoor space 3 by a sick animal 21, and wherein the second air 82 comprising the attenuated airborne pathogen 72 is provided to a (healthy) animal 20 (of the same species) in the indoor space, which may trigger and train the immune system of the animal 20, thereby providing resistance to subsequent exposure to the airborne pathogen 71.

Further, the system may be applied in a livestock health management system, especially with a UVC light setting selected from a set of options and based on an ongoing outbreak with a specific airborne pathogen 71. The system 1000, especially the control system 300, may be configured to anticipate on a possible disease outbreak among animals 20 in the indoor space 3, such as in a stable, such that an early infection/infectious animal 21 might serve the purpose as a fast immunity provider for the other animals 20.

Hence, in the depicted embodiment, the indoor space 3 may be an indoor space for (hosting) one or more of poultry and livestock.

In further embodiments, the airborne pathogen 71 may comprise one or more of a virus, bacteria, and fungi. In particular, the airborne pathogen 71 may be a respiratory pathogen i.e., a pathogen that enters the host body via the respiratory track. Hence, in specific embodiments, the airborne pathogen 71 may comprise a respiratory virus.

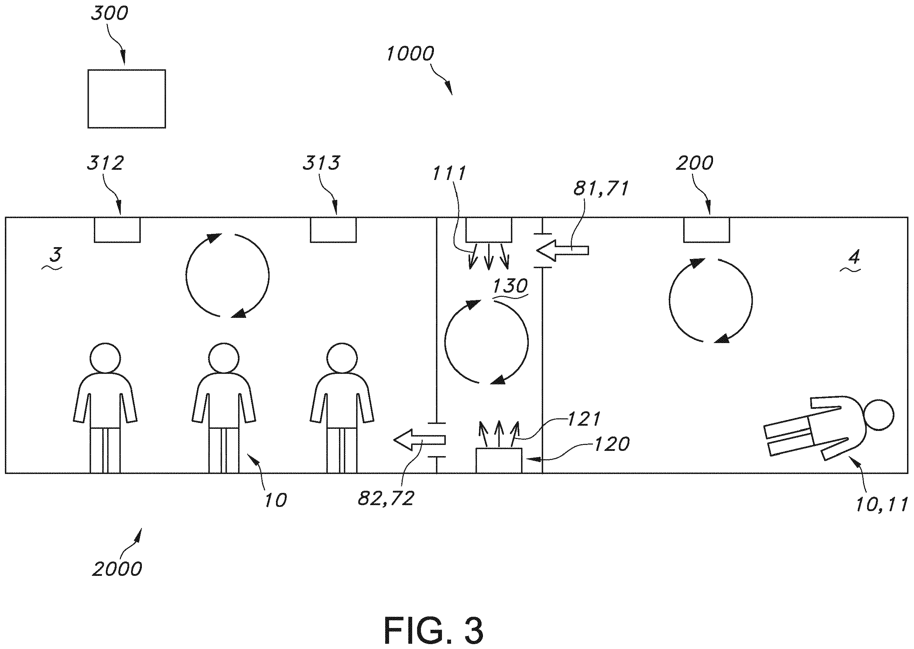

Fig. 3 schematically depicts a further embodiment of the arrangement 2000. In the depicted embodiment, the system 1000 comprises a second indoor space 4, wherein the second indoor space 4 is configured to host a human patient 11, especially a human patient 11 having a respiratory disease. In such embodiments, the system 1000 may especially be configured to retrieve the first air 81 from the second indoor space 4.

In the depicted embodiment, one or more of the (first) indoor space 3 and the second indoor space 4 may comprise the pathogen sensor 312; here especially the indoor space 3. In further embodiments, the system 1000, especially the control system, may be configured to control in an operational mode the treatment mode in dependence of one or more of the pathogen sensor signal, and information from an external database 302.

In further embodiments, the second indoor space 4 may comprise the pathogen sensor 312. Thereby, the system 1000 may confirm the presence, especially the load, of the airborne pathogen 71 in the first air 81.

In further embodiments, the enclosure 130 may comprise the pathogen sensor 312. Thereby, the system 1000 may determine the level of disinfection, especially prior to providing the second air 82 to the indoor space 3.

In the depicted embodiment, the system 1000 further comprises a second light generating device 120 configured to generate second radiation 121. In an operational mode of the system 1000 the second radiation 121 may have a second spectral power distribution of which at least 90% of the spectral power in the 100-300 nm wavelength range is in the 100-255 nm wavelength range. Hence, the second light generating device 120 may further contribute to attenuating the airborne pathogen 71. However, the second light generating device 120 may particularly be suitable for killing the airborne pathogen 71.

Hence, in embodiments, the second light generating device 120 may especially be configured to provide second radiation 121 different from the first radiation 111. For instance, in further embodiments, the first radiation 111 may have a spectral power distribution of which at least 90% of the spectral power in the 100 - 300 nm wavelength range is in the 255-300 nm wavelength range, whereas the second radiation may have a spectral power distribution of which at least 90% of the spectral power in the 100 - 300 nm wavelength range is in the 100 -255 wavelength range. Thereby, the system 1000, especially the control system 300, may have additional control over the attenuation and/or killing of the airborne pathogen 71. In particular, the system 1000, especially the control system 300, may be configured to control the second light generating device 120 to provide the second radiation when an abundance of the airborne pathogen 71 (in the first air) exceeds a predefined threshold.

Specifically, in the depicted embodiment, the arrangement 2000 may comprise a second indoor space 4 where there is guaranteed airborne viral load (as expelled by the human patient 11 that resides there), and a (first) indoor space 3 where people are exposed to attenuated (or "inactivated") airborne pathogen (that they inhale). This inactivated airborne virus may now acts as Protein-based respiratory vaccine. Airflows between the spaces may be (actively) steered to first deactivate the virus particles using first radiation 111 and next transferring the attenuated virus towards the recipient humans 10. In the depicted embodiment, the arrangement 2000, especially the system 1000, further comprises an upper air disinfection device 200 that may be configured to deactivate (other) airborne pathogens, such as (other) airborne viruses, and (at least partially) prevent (further) infections/spreading in the patient ward.

As indicated above, the second radiation 121 may be provided to address an overabundance of the pathogen 71. Hence, in general, the second radiation source 120 may not provide the second radiation 121.

Fig. 1-3 further schematically depict the method for distributing an attenuated airborne pathogen 72 in an indoor space 3 for humans 10 or animals 20. The method may comprise: subjecting in a treatment mode first air 81 comprising an airborne pathogen 71 to first radiation 111 to attenuate the airborne pathogen 71 to provide second air 82 with the attenuated airborne pathogen 72. The method may further comprise introducing the second air 82 with the attenuated airborne pathogen 72 in the indoor space 3.

The term "plurality" refers to two or more. Furthermore, the terms "a plurality of" and "a number of" may be used interchangeably.

The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%. Moreover, the terms "about" and "approximately" may also relate to 90% or higher, such as 95% or higher, especially 99% or higher, even more especially 99.5% or higher, including 100%. For numerical values it is to be understood that the terms "substantially", "essentially", "about", and "approximately" may also relate to the range of 90% - 110%, such as 95%-105%, especially 99%-101% of the values(s) it refers to.

The term "comprise" also includes embodiments wherein the term "comprises" means "consists of".

The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices, apparatus, or systems may herein amongst others be described during operation. As will be clear to the person skilled in the art, the invention is not limited to methods of operation, or devices, apparatus, or systems in operation.

The term "further embodiment" and similar terms may refer to an embodiment comprising the features of the previously discussed embodiment, but may also refer to an alternative embodiment.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", "include", "including", "contain", "containing" and the like are to be construed in an inclusive sense as opposed to an exclusive or exhaustive sense; that is to say, in the sense of "including, but not limited to".

The article "a" or "an" preceding an element does not exclude the presence of a plurality of such elements.

The invention may be implemented by means of hardware comprising several distinct elements, and by means of a suitably programmed computer. In a device claim, or an apparatus claim, or a system claim, enumerating several means, several of these means may be embodied by one and the same item of hardware. The mere fact that certain measures are recited in mutually different dependent claims does not indicate that a combination of these measures cannot be used to advantage.

The invention also provides a control system that may control the device, apparatus, or system, or that may execute the herein described method or process. Yet further, the invention also provides a computer program product, when running on a computer which is functionally coupled to or comprised by the device, apparatus, or system, controls one or more controllable elements of such device, apparatus, or system.

The invention further applies to a device, apparatus, or system comprising one or more of the characterizing features described in the description and/or shown in the attached drawings. The invention further pertains to a method or process comprising one or more of the characterizing features described in the description and/or shown in the attached drawings. Moreover, if a method or an embodiment of the method is described being executed in a device, apparatus, or system, it will be understood that the device, apparatus, or system is suitable for or configured for (executing) the method or the embodiment of the method, respectively.

The various aspects discussed in this patent can be combined in order to provide additional advantages. Further, the person skilled in the art will understand that embodiments can be combined, and that also more than two embodiments can be combined. Furthermore, some of the features can form the basis for one or more divisional applications.

## Claims

1. A system (1000) for distributing an attenuated airborne pathogen (72) in an indoor space (3) for humans (10) or animals (20), wherein the system (1000) is configured to (a) subject in a treatment mode first air (81) comprising an airborne pathogen (71) to first radiation (111) to attenuate the airborne pathogen (71) to provide second air (82) with the attenuated airborne pathogen (72), and to (b) introduce the second air (82) in the indoor space (3); wherein a spectral power distribution of the first radiation (111) is controllable in at least part of a wavelength range of 100-380 nm, wherein the system (1000) comprises a control system (300) configured to control the spectral power distribution of the first radiation (111) of the treatment mode in dependence of the airborne pathogen (71) to be attenuated, and
the system (1000) further comprising an indoor climate sensor (313) configured to sense an indoor climate parameter in the indoor space (3) and to provide a related indoor climate sensor signal, wherein the indoor climate parameter is selected from the group comprising temperature, humidity, airflow, airborne particle size, airborne particle size distribution and air composition, and wherein the system (1000) is configured to control in an operational mode the treatment mode in dependence of the indoor climate sensor signal by controlling one or more of (i) the spectral power distribution of the first radiation (111), (ii) an intensity of the first radiation (111) and (iii) an exposure time of the first air (81) to the first radiation.

2. The system (1000) according to claim 1, wherein the airborne pathogen (71) comprises a respiratory virus.

3. The system (1000) according to any one of the preceding claims, wherein the airborne pathogen (71) comprises a core part comprising genetic material and an outer part comprising outer part material, wherein the outer part material comprises one or more of proteins, lipids and polysaccharides, wherein a spectral power distribution of the first radiation (111) is selected such that in a part of the wavelength range of 100-380 nm a ratio between absorption of radiation by the genetic material and absorption of radiation by the outer part material in that part of the wavelength range is at least 2.

4. The system (1000) according to any one of the preceding claims, wherein the system (1000) comprises a radiation source (110) configured to generate the first radiation (111), wherein the first radiation (111) has one or more wavelengths selected from the wavelength range of 100-300 nm.

5. The system (1000) according to claim 4, wherein in an operational mode of the system (1000) the first radiation (111) has a first spectral power distribution of which at least 90% of the spectral power in the 100-300 nm wavelength range is in the 255-300 nm wavelength range.

6. The system (1000) according to any one of the preceding claims 4-5, wherein the spectral power distribution of the first radiation (111) and the intensity of the first radiation (111) are selected to provide at least a 1-log reduction of the airborne pathogen (71).

7. The system (1000) according to any one of the preceding claims, further comprising a radiation sensor (311), wherein the radiation sensor (311) is configured to detect third radiation (131) having a third wavelength in the range of 550-680 nm, and wherein the system (1000) is configured to control in an operational mode the treatment mode in dependence of the third radiation (131).

8. The system (1000) according to any one of the preceding claims, further comprising a pathogen sensor (312) configured to sense the airborne pathogen (71) in the indoor space (3) and to provide a related pathogen sensor signal, and wherein the system (1000) is configured to control in an operational mode the treatment mode in dependence of the pathogen sensor signal.

9. The system (1000) according to any one of the preceding claims, the system (1000) being configured to select the first radiation (111) based on the composition of the airborne particles in the first air (81).

10. The system (1000) according to any one of the preceding claims, having a controllable spectral power distribution of the first radiation (111) within at least part of a wavelength range of 255-300 nm, wherein the control system (300) is configured to control the spectral power distribution in dependence of one or more of an input signal of a user interface, a sensor signal, a timer, and external database information.

11. An arrangement (2000) comprising (a) the system (1000) according to any one of the preceding claims and (b) an indoor space (3), wherein the system (1000) is configured to (a) subject in the treatment mode first air (81) comprising the airborne pathogen (71) to first radiation (111) to attenuate the airborne pathogen (71) to provide second air (82) with the attenuated airborne pathogen (72), and to (b) introduce the second air (82) with the attenuated airborne pathogen (72) in the indoor space (3).

12. The arrangement (2000) according to claim 11, comprising a second indoor space (4), wherein the second indoor space (4) is configured to host a human patient (11) having a respiratory disease, and wherein the system (1000) is configured to retrieve the first air (81) from the second indoor space (4).

13. The arrangement (2000) according to claim 12, wherein one or more of the indoor space (3) and the second indoor space (4) comprises the pathogen sensor (312) according to claim 8, and wherein the system (1000) is configured to control in an operational mode the treatment mode in dependence of one or more of (i) the pathogen sensor signal, and (ii) the external database information according to claim 10.

14. The arrangement (2000) according claim 11, wherein the indoor space (3) is an indoor space for one or more of poultry and livestock.

15. A method for distributing an attenuated airborne pathogen (72) in an indoor space (3) for humans or animals using the system (1000) according to claim 1 , the method comprising: (a) subjecting in a treatment mode first air (81) comprising an airborne pathogen (71) to first radiation (111) to attenuate the airborne pathogen (71) to provide second air (82) with the attenuated airborne pathogen (72), and (b) introducing the second air (82) with the attenuated airborne pathogen (72) in the indoor space (3).

## Patentansprüche

1. System (1000) zum Verteilen eines abgeschwächten aerogenen Krankheitserregers (72) in einem Innenraum (3) für Menschen (10) oder Tiere (20), wobei das System (1000) konfiguriert ist, um (a) in einem Behandlungsmodus erste Luft (81), umfassend einen aerogenen Krankheitserreger (71), einer ersten Strahlung (111) auszusetzen, um den aerogenen Krankheitserreger (71) abzuschwächen, um zweite Luft (82) mit dem abgeschwächten aerogenen Krankheitserreger (72) zu versehen, und um (b) die zweite Luft (82) in den Innenraum (3) einzuführen; wobei eine spektrale Leistungsverteilung der ersten Strahlung (111) in mindestens einem Teil eines Wellenlängenbereichs von 100-380 nm steuerbar ist, wobei das System (1000) ein Steuersystem (300) umfasst, das konfiguriert ist, um die spektrale Leistungsverteilung der ersten Strahlung (111) des Behandlungsmodus in Abhängigkeit von dem abzuschwächenden aerogenen Krankheitserreger (71) zu steuern, und
das System (1000) ferner umfassend einen Innenraumklimasensor (313), der konfiguriert ist, um einen Innenraumklimaparameter in dem Innenraum (3) zu erfassen und ein zugehöriges Innenraumklimasensorsignal bereitzustellen, wobei der Innenraumklimaparameter aus der Gruppe ausgewählt ist, umfassend Temperatur, Feuchtigkeit, Luftstrom, Größe der aerogenen Partikel, Größenverteilung der aerogenen Partikel und Luftzusammensetzung, und wobei das System (1000) konfiguriert ist, um in einem Betriebsmodus den Behandlungsmodus in Abhängigkeit von dem Innenraumklimasensorsignal durch Steuern von einem oder mehreren von (i) der spektralen Leistungsverteilung der ersten Strahlung (111), (ii) einer Intensität der ersten Strahlung (111) und (iii) einer Aussetzungszeit der ersten Luft (81) der ersten Strahlung zu steuern.

2. System (1000) nach Anspruch 1, wobei der aerogene Krankheitserreger (71) einen Atemwegsvirus umfasst.

3. System (1000) nach einem der vorstehenden Ansprüche, wobei der aerogene Krankheitserreger (71) einen Kernteil, umfassend genetisches Material, und einen Außenteil umfasst, umfassend Außenteilmaterial, wobei das Außenteilmaterial eines oder mehrere von Proteinen, Lipiden und Polysacchariden umfasst, wobei eine spektrale Leistungsverteilung der ersten Strahlung (111) derart ausgewählt ist, dass in einem Teil des Wellenlängenbereichs von 100-380 nm ein Verhältnis zwischen der Absorption von Strahlung durch das genetische Material und der Absorption von Strahlung durch das Außenteilmaterial in diesem Teil des Wellenlängenbereichs mindestens 2 beträgt.

4. System (1000) nach einem der vorstehenden Ansprüche, wobei das System (1000) eine Strahlungsquelle (110) umfasst, die konfiguriert ist, um die erste Strahlung (111) zu erzeugen, wobei die erste Strahlung (111) eine oder mehrere Wellenlängen aufweist, die aus dem Wellenlängenbereich von 100-300 nm ausgewählt sind.

5. System (1000) nach Anspruch 4, wobei in einem Betriebsmodus des Systems (1000), die erste Strahlung (111) eine erste spektrale Leistungsverteilung aufweist, bei der mindestens 90 % der spektralen Leistung in dem Wellenlängenbereich von 100-300 nm in dem Wellenlängenbereich von 255-300 nm liegt.

6. System (1000) nach einem der vorstehenden Ansprüche 4 bis 5, wobei die spektrale Leistungsverteilung der ersten Strahlung (111) und die Intensität der ersten Strahlung (111) ausgewählt sind, um eine Reduzierung des aerogenen Krankheitserregers (71) um mindestens 1-log bereitzustellen.

7. System (1000) nach einem der vorstehenden Ansprüche, ferner umfassend einen Strahlungssensor (311), wobei der Strahlungssensor (311) konfiguriert ist, um eine dritte Strahlung (131) zu erkennen, die eine dritte Wellenlänge in dem Bereich von 550-680 nm aufweist, und wobei das System (1000) konfiguriert ist, um in einem Betriebsmodus den Behandlungsmodus in Abhängigkeit von der dritten Strahlung (131) zu steuern.

8. System (1000) nach einem der vorstehenden Ansprüche, ferner umfassend einen Krankheitserregersensor (312), der konfiguriert ist, um den aerogenen Krankheitserreger (71) in dem Innenraum (3) zu erfassen und um ein zugehöriges Krankheitserregersensorsignal bereitzustellen, und wobei das System (1000) konfiguriert ist, um in einem Betriebsmodus den Behandlungsmodus in Abhängigkeit von dem Erregersensorsignal zu steuern.

9. System (1000) nach einem der vorstehenden Ansprüche, wobei das System (1000) konfiguriert ist, um die erste Strahlung (111) basierend auf der Zusammensetzung der aerogenen Partikel in der ersten Luft (81) auszuwählen.

10. System (1000) nach einem der vorstehenden Ansprüche, das eine steuerbare spektrale Leistungsverteilung der ersten Strahlung (111) innerhalb von mindestens einem Teil eines Wellenlängenbereichs von 255-300 nm aufweist, wobei das Steuersystem (300) konfiguriert ist, um die spektrale Leistungsverteilung in Abhängigkeit von einem oder mehreren von einem Eingangssignal einer Benutzerschnittstelle, einem Sensorsignal, einem Zeitgeber und externen Datenbankinformationen zu steuern.

11. Anordnung (2000), umfassend (a) das System (1000) nach einem der vorstehenden Ansprüche und (b) einen Innenraum (3), wobei das System (1000) konfiguriert ist, um (a) in dem Behandlungsmodus erste Luft (81), umfassend den aerogenen Krankheitserreger (71), einer ersten Strahlung (111) auszusetzen, um den aerogenen Krankheitserreger (71) abzuschwächen, um zweite Luft (82) mit dem abgeschwächten aerogen Krankheitserreger (72) zu versehen, und um (b) die zweite Luft (82) mit dem abgeschwächten aerogenen Krankheitserreger (72) in den Innenraum (3) einzuführen.

12. Anordnung (2000) nach Anspruch 11, umfassend einen zweiten Innenraum (4), wobei der zweite Innenraum (4) konfiguriert ist, um einen menschlichen Patienten (11) aufzunehmen, der eine Atemwegserkrankung aufweist, und wobei das System (1000) konfiguriert ist, um die erste Luft (81) aus dem zweiten Innenraum (4) abzusaugen.

13. Anordnung (2000) nach Anspruch 12, wobei einer oder mehrere der Innenräume (3) und der zweite Innenraum (4) den Krankheitserregersensor (312) nach Anspruch 8 umfassen, und wobei das System (1000) konfiguriert ist, um in einem Betriebsmodus den Behandlungsmodus in Abhängigkeit von einem oder mehreren von (i) dem Krankheitserregersensorsignal und (ii) den externen Datenbankinformationen nach Anspruch 10 zu steuern.

14. Anordnung (2000) nach Anspruch 11, wobei der Innenraum (3) ein Innenraum für eines oder mehrere von Geflügel und/oder Vieh ist.

15. Verfahren zum Verteilen eines abgeschwächten, aerogenen Krankheitserregers (72) in einem Innenraum (3) für Menschen oder Tiere unter Verwendung des Systems (1000) nach Anspruch 1, das Verfahren umfassend: (a) Aussetzen erster Luft (81), umfassend einen aerogenen Krankheitserreger (71), einer ersten Strahlung (111) in einem Behandlungsmodus, um den aerogenen Krankheitserreger (71) abzuschwächen und um zweite Luft (82) mit dem abgeschwächten aerogenen Krankheitserreger (72) zu versehen, und (b) Einführen der zweiten Luft (82) mit dem abgeschwächten aerogenen Krankheitserreger (72) in den Innenraum (3).

## Revendications

1. Système (1000) de distribution d'un agent pathogène aéroporté atténué (72) dans un espace intérieur (3) pour les humains (10) ou les animaux (20), dans lequel le système (1000) est configuré pour (a) soumettre dans un mode de traitement le premier air (81) comprenant un agent pathogène aéroporté (71) à un premier rayonnement (111) pour atténuer l'agent pathogène aéroporté (71) afin de fournir un second air (82) contenant l'agent pathogène aéroporté atténué (72), et pour (b) introduire le second air (82) dans l'espace intérieur (3) ; dans lequel une distribution de puissance spectrale du premier rayonnement (111) est commandable dans au moins une partie d'une plage de longueur d'onde de 100-380 nm, dans lequel le système (1000) comprend un système de commande (300) configuré pour commander la distribution de puissance spectrale du premier rayonnement (111) du mode de traitement en fonction de l'agent pathogène aéroporté (71) à atténuer, et
le système (1000) comprenant en outre un capteur de climat intérieur (313) configuré pour détecter un paramètre de climat intérieur dans l'espace intérieur (3) et pour fournir un signal de capteur de climat intérieur correspondant, dans lequel le paramètre de climat intérieur est choisi parmi le groupe comprenant la température, l'humidité, le débit d'air, la taille des particules aéroportées, la distribution de la taille des particules aéroportées et la composition de l'air, et dans lequel le système (1000) est configuré pour commander dans un mode opérationnel le mode de traitement en fonction du signal du capteur de climat intérieur en commandant l'un ou plusieurs parmi (i) la distribution de puissance spectrale du premier rayonnement (111), (ii) une intensité du premier rayonnement (111) et (iii) un temps d'exposition du premier air (81) au premier rayonnement.

2. Système (1000) selon la revendication 1, dans lequel l'agent pathogène aéroporté (71) comprend un virus respiratoire.

3. Système (1000) selon l'une quelconque des revendications précédentes, dans lequel l'agent pathogène aéroporté (71) comprend une partie centrale comprenant du matériel génétique et une partie extérieure comprenant du matériel de partie extérieure, dans lequel le matériel de partie extérieure comprend l'un ou plusieurs parmi les protéines, les lipides et les polysaccharides, dans lequel une distribution de puissance spectrale du premier rayonnement (111) est choisie de telle sorte que, dans une partie de la plage de longueur d'onde de 100-380 nm, un rapport entre l'absorption du rayonnement par le matériel génétique et l'absorption du rayonnement par le matériel de partie extérieure dans cette partie de la plage de longueur d'onde est d'au moins 2.

4. Système (1000) selon l'une quelconque des revendications précédentes, dans lequel le système (1000) comprend une source de rayonnement (110) configurée pour générer le premier rayonnement (111), dans lequel le premier rayonnement (111) a une ou plusieurs longueurs d'onde choisies à partir de la plage de longueur d'onde de 100-300 nm.

5. Système (1000) selon la revendication 4, dans lequel, dans un mode opérationnel du système (1000), le premier rayonnement (111) a une première distribution de puissance spectrale dont au moins 90 % de la puissance spectrale dans la plage de longueur d'onde de 100-300 nm se situe dans la plage de longueur d'onde de 255-300 nm.

6. Système (1000) selon l'une quelconque des revendications précédentes 4-5, dans lequel la distribution de puissance spectrale du premier rayonnement (111) et l'intensité du premier rayonnement (111) sont choisies pour fournir au moins une réduction de 1 log de l'agent pathogène aéroporté (71).

7. Système (1000) selon l'une quelconque des revendications précédentes, comprenant en outre un capteur de rayonnement (311), dans lequel le capteur de rayonnement (311) est configuré pour détecter un troisième rayonnement (131) ayant une troisième longueur d'onde dans la plage de 550-680 nm, et dans lequel le système (1000) est configuré pour commander dans un mode opérationnel le mode de traitement en fonction du troisième rayonnement (131).

8. Système (1000) selon l'une quelconque des revendications précédentes, comprenant en outre un capteur d'agent pathogène (312) configuré pour détecter l'agent pathogène aéroporté (71) dans l'espace intérieur (3) et pour fournir un signal de capteur d'agent pathogène correspondant, et dans lequel le système (1000) est configuré pour commander dans un mode opérationnel le mode de traitement en fonction du signal de capteur d'agent pathogène.

9. Système (1000) selon l'une quelconque des revendications précédentes, le système (1000) étant configuré pour choisir le premier rayonnement (111) sur la base de la composition des particules aéroportées dans le premier air (81).

10. Système (1000) selon l'une quelconque des revendications précédentes, ayant une distribution de puissance spectrale commandable du premier rayonnement (111) dans au moins une partie d'une plage de longueur d'onde de 255-300 nm, dans lequel le système de commande (300) est configuré pour commander la distribution de puissance spectrale en fonction d'un ou plusieurs parmi un signal d'entrée d'une interface utilisateur, un signal de capteur, une minuterie et des informations de base de données externe.

11. Dispositif (2000) comprenant (a) le système (1000) selon l'une quelconque des revendications précédentes et (b) un espace intérieur (3), dans lequel le système (1000) est configuré pour (a) soumettre, dans le mode de traitement, le premier air (81) comprenant l'agent pathogène aéroporté (71) à un premier rayonnement (111) pour atténuer l'agent pathogène aéroporté (71) afin de fournir un second air (82) contenant l'agent pathogène aéroporté atténué (72), et pour (b) introduire le second air (82) contenant l'agent pathogène aéroporté atténué (72) dans l'espace intérieur (3).

12. Agencement (2000) selon la revendication 11, comprenant un second espace intérieur (4), dans lequel le second espace intérieur (4) est conçu pour accueillir un patient humain (11) ayant une maladie respiratoire, et dans lequel le système (1000) est configuré pour récupérer le premier air (81) du second espace intérieur (4).

13. Agencement (2000) selon la revendication 12, dans lequel l'un ou plusieurs parmi l'espace intérieur (3) et le second espace intérieur (4) comprend le capteur d'agent pathogène (312) selon la revendication 8, et dans lequel le système (1000) est configuré pour commander dans un mode opérationnel le mode de traitement en fonction d'un ou plusieurs parmi (i) le signal de capteur d'agent pathogène, et (ii) les informations de base de données externe selon la revendication 10.

14. Agencement (2000) selon la revendication 11, dans lequel l'espace intérieur (3) est un espace intérieur pour l'un ou plusieurs parmi la volaille et le bétail.

15. Procédé de distribution d'un agent pathogène aéroporté atténué (72) dans un espace intérieur (3) pour les humains ou les animaux à l'aide du système (1000) selon la revendication 1, le procédé comprenant : (a) la soumission, dans un mode de traitement, du premier air (81) comprenant un agent pathogène aéroporté (71) à un premier rayonnement (111) pour atténuer l'agent pathogène aéroporté (71) afin de fournir un second air (82) contenant l'agent pathogène aéroporté atténué (72), et (b) l'introduction du second air (82) contenant l'agent pathogène aéroporté atténué (72) dans l'espace intérieur (3).
